# FASCICULE DE BREVET EUROPEEN

(11) **EP 0 959 924 B1**
(45) Date de publication et mention de la délivrance du brevet: **21.06.2006**
(21) Numéro de dépôt: 97944679.6
(22) Date de dépôt: 28.10.1997
(51) Int. Cl.: A61M 5/50

(54) **SERINGUE MEDICALE**
MEDIZINISCHE SPRITZE
MEDICAL SYRINGE

(30) Priorité: 27.11.1996 CH 292296
(43) Date de publication de la demande: 01.12.1999
(73) Titulaire: SSE System Services Establishment, 9490 Vaduz (LI)
(72) Inventeur: GRIMM, Edgardo, CH-2000 Neuchâtel (CH); MORO, Olinto, CH-1006 Lausanne (CH)
(74) Mandataire: Ganguillet, Cyril
(86) Numéro de dépôt international: PCT/CH1997/000410
(87) Numéro de publication internationale: WO 1998/023316

(56) Documents cités:
- EP-A- 0 345 159
- WO-A-90/03817
- FR-A- 2 298 340
- FR-A- 2 606 643
- GB-A- 2 207 054

## Description

La présente invention concerne une seringue médicale comportant des éléments constructifs autorisant une injection mais interdisant l'utilisation de la même seringue pour toute injection subséquente. En bref, il s'agit d'une seringue à usage unique autodestructive, sans aucune manipulation particulière de l'opérateur.

Certes, pour des raisons évidentes, les milieux hospitaliers et médicaux utilisent de longue date des seringues jetables qui ne servent qu'une fois. Cependant, ces milieux ne sont pas les seuls à faire usage de seringues et confier l'unicité de l'usage à la seule discipline de l'utilisateur n'est de loin pas suffisant dans le monde réel. Il est par exemple connu, selon des études de l'OMS, que dans les pays en voie de développement, les seringues dites jetables sont souvent réutilisées jusqu'à 50 fois.

C'est la raison pour laquelle de nombreuses propositions ont été faites pour réaliser des seringues qui, par la manière dont elles sont conçues ou construites, interdisent physiquement une deuxième utilisation.

Le choix des moyens à mettre en oeuvre dans ce but est fort limité. En fait il n'y a guère qu'une seule voie, à savoir munir la chambre de la seringue, en principe la tête du piston, d'un organe d'obturation provisoire qui assure l'étanchéité de la chambre lors de la première injection, mais qui est désactivé définitivement lorsque la première injection est accomplie.

L'expression la plus simple de cette construction est décrite dans la demande de brevet FR-A-2,606,643 concernant une seringue à embout excentré, où la tête du piston est pourvue d'un orifice, bouché à l'origine par un bouchon amovible qui est éjecté hors de la chambre lorsqu'il rencontre un pointeau alors que le piston arrive en fin de course et que la chambre parvient à son volume minimum.

Des propositions plus sophistiquées ont également été faites, recourant notamment à une membrane portée par le piston, membrane qui est soit déplacée par la pression du liquide, soit percée à la fin de la première injection.

Le document EP 0345159 décrit une seringue à injection unique comportant un corps de seringue cylindrique à la base duquel peut être fixée une aiguille de prélèvement et/ou d'injection, et un piston coulissant à l'intérieur du corps de seringue dont l'extrémité porte un joint glissant de façon étanche sur la paroi intérieure de la seringue. Le joint comporte au moins un organe susceptible d'être déformé ou déplacé à partir de sa position d'équilibre sous l'action de la pression qu'exerce le liquide contenu dans la seringue lors de la phase d'injection et le piston coulissant est associé audit joint par l'intermédiaire de pièces qui réagissent à la déformation ou au déplacement dudit organe et qui rendent le joint inopérant pour un nouveau remplissage à l'issue de l'injection.

Le document WO 90/03817 décrit un dispositif d'injection comprenant un cylindre d'injection contenant un piston relié à une tige de piston plus longue que le cylindre d'injection. Des moyens d'accouplement permettent de monter une aiguille sur le cylindre d'injection. Pour éviter une réutilisation du dispositif d'injection des moyens pour provoquer une ouverture irréversible dans le piston sont montés dans la tige de piston ou dans le cylindre d'injection.

Le document GB 2207054 décrit une seringue comprenant un cylindre sur lequel peut s'adapter une aiguille, un piston coulissant dans le cylindre, avec des moyens pour rendre étanche une chambre volumétrique à l'avant du cylindre. Au piston sont associés des moyens pour rendre inefficaces les moyens d'étanchéité après le premier usage de la seringue. Ceux-ci comprennent un insert dans les moyens d'étanchéité, qui est éjecté lorsqu'une pression négative s'exerce sur le piston. Ils peuvent également comprendre une pièce perçant un diaphragme du piston en fin de course.

Le document FR 2298340 décrit une seringue comportant un corps cylindrique et un piston dont l'extrémité comporte une paroi déchirable qui vient en regard d'une pointe portée par le fond du corps cylindrique perçant la paroi déchirable lors de l'enfoncement du piston de la seringue dans le corps.

Le nombre et la variété des propositions de l'art antérieur pourrait laisser penser que le tour du problème a été fait. Cependant, tel n'est pas le cas. En effet, il ne suffit pas de concevoir une seringue qui puisse fonctionner selon ce principe ou l'une de ses nombreuses variantes, encore faut-il pouvoir la produire selon un processus industriel qui autorise un coût extrêmement faible. A cet égard, il convient de rappeler que la fabrication industrielle de seringues doit se plier à deux impératifs. En premier lieu, les quantités sont proprement astronomiques et se chiffrent en milliards d'unités pour atteindre un coût unitaire simplement décent dans le contexte. En second lieu, les seringues doivent être conditionnées en emballage stérile de qualité, cela malgré le débit énorme de la fabrication. C'est sans aucun doute pour ne pas permettre le respect de ces deux conditions que les propositions selon l'art antérieur n'ont débouché sur aucune production significative, alors que le besoin est tout à fait connu et tout à fait pressant.

C'est ainsi sous cet angle que la critique des propositions antérieures doit être faite car il s'agit bien de constater que ces propositions ne répondent pas aux besoins tels que le monde les connaît dans la réalité. Pour qu'une seringue à usage unique réponde vraiment au besoin, il est nécessaire que son prix de revient unitaire soit au moins comparable à celui d'une seringue jetable traditionnelle, mais il est aussi nécessaire que sa fiabilité soit égale. Il serait en effet ridicule de consentir des efforts de production importants si le caractère physiquement unique de l'usage n'est pas garanti et qu'il reste aléatoire comme c'est les cas pour les propositions qui ont été faites jusqu'ici.

Le recours à une membrane est disqualifié en milieu industriel car s'agissant par nécessité d'objets en matière plastique, l'élasticité est un paramètre proprement incontrôlable, en particulier dans le temps. On ne peut donc garantir des propriétés stables et de ce fait on s'expose soit à l'impossibilité d'exécuter proprement la première injection, soit à l'incertitude d'interdire dans tous les cas la deuxième injection. On peut relever par exemple que dans le cas de la seringue proposée dans le brevet européen 0345159, du fait de la présence de la membrane souple à l'extrémité du piston, la mise en action du dispositif est commandée par la nature de la pression que l'on exerce sur le liquide à injecter. La moindre chute de pression peut interrompre pour toujours l'injection en cours.

Le recours à un bouchon éjecté est également à écarter car s'agissant d'une pièce de plastique insérée dans une autre, on ne peut sérieusement prétendre maîtriser l'instant auquel elles se dissocient. En d'autres termes le bouchon peut parfaitement sauter durant l'injection sous la seule pression du liquide, de même qu'il peut être déjà éjecté dans l'autre direction lors de l'aspiration du liquide dans la seringue. En tout cas, personne ne peut garantir qu'il ne sautera pas et cela est rédhibitoire.

Le but de la présente invention est ainsi de proposer une seringue à usage unique dont les éléments constitutifs, et les propriétés qu'ils ont en combinaison les uns avec les autres, autorisent d'une part une production industrielle économique et fiable tout en garantissant d'autre part, par les propriétés mécaniques contrôlables des dits éléments, que l'usage sera vraiment unique.

A cet effet, la présente invention concerne une seringue médicale comprenant un corps tubulaire, dans lequel coulisse un piston comportant une ouverture, une pièce d'obturation obturant ladite ouverture avant usage de la seringue, cette pièce d'obturation étant agencée pour coopérer, lorsque le piston arrive en fin de course, avec une contre-pièce solidaire du corps tubulaire de la seringue, de façon à chasser ladite pièce d'obturation hors de l'ouverture, de manière que, après utilisation de la seringue, la pièce d'obturation n'obture définitivement plus l'ouverture, de sorte que la seringue n'est pas réutilisable. Le piston est agencé de façon à constituer une chambre interne à l'intérieur de laquelle est logée la pièce d'obturation, l'ensemble étant agencé de façon à imposer une course prédéterminée à la pièce d'obturation, sans recours à un élément déformante, la pièce d'obturation comportant une tête d'obturation engagée dans l'ouverture et au moins un organe agencé pour empêcher son retour à sa position d'obturation, ledit organe s'appuyant, avant injection, contre la paroi interne de ladite chambre interne, de façon à engendrer une force de friction qui s'ajoute à celle qu'exerce le bord de l'ouverture sur la tête d'obturation, de façon que la pression du liquide s'exerçant sur la tête d'obturation lors de l'injection est insuffisante pour chasser la tête d'obturation de l'ouverture, toutes les pièces de la seringue étant conformées de façon à pouvoir être produite par moulage et montées en série automatiquement par emboîtage axial

Selon une forme d'exécution de la seringue, la pièce d'obturation comporte à sa partie postérieure au moins une ailette tolérant une déformation élastique dont l'extrémité libre est orientée en direction du fond du corps de la seringue et coopère avec la paroi de la chambre interne, la paroi de la chambre interne étant conformée de façon à permettre à l'extrémité libre de l'ailette de coulisser contre la paroi de la chambre interne lorsque la pièce d'obturation se déplace à l'intérieur de la chambre après avoir buté contre la contre-pièce, mais à empêcher ensuite le coulissement de l'extrémité libre de l'ailette en sens inverse.

La chambre interne peut comporter un premier espace dont les dimensions sont choisies de façon à maintenir l'ailette repliée le long de la pièce d'obturation lorsque le tête d'obturation de la pièce d'obturation obture l'ouverture, et une second espace dont les dimensions sont choisies de façon à permettre le déploiement de l'ailette lorsque la pièce d'obturation a été déplacée à l'intérieur du piston après avoir buté sur la contre-pièce.

Selon une forme d'exécution préférentielle, la contre-pièce comporte au moins un organe faisant saillie par rapport au fond du corps tubulaire de la seringue.

D'autres caractéristiques, buts et avantages de l'invention apparaîtront plus clairement à la lumière de la description d'une forme d'exécution de la seringue et des variantes données ci-après à titre d'exemple et illustrées par les dessins annexés dans lesquels:
- la figure 1 est une coupe axiale de la partie antérieure d'un premier mode de réalisation de la seringue, telle qu'elle se présente avant ou au cours de l'injection;
- la figure 2 est une coupe analogue à celle de la figure 1 de la partie antérieure de la même seringue, telle qu'elle se présente à la suite d'une tentative de réutilisation;
- la figure 3 est une coupe axiale de la partie antérieure d'un deuxième mode de réalisation de la seringue, telle qu'elle se présente avant ou au cours de l'injection;
- la figure 4 est une coupe analogue à celle de la figure 3 de la partie antérieure de la même seringue, telle qu'elle se présente à la suite d'une tentative de réutilisation;
- les figures 5 à 7 sont des coupes axiales partielles de l'extrémité antérieure de la seringue présentant trois variantes de contre-pièce servant de butée à la pièce d'obturation; et
- la figure 8 est une coupe axiale de la partie antérieure d'une variante d'exécution de la seringue, la demi-coupe de droite représentant la seringue avant injection et la demi-coupe de gauche représentant la seringue après injection.

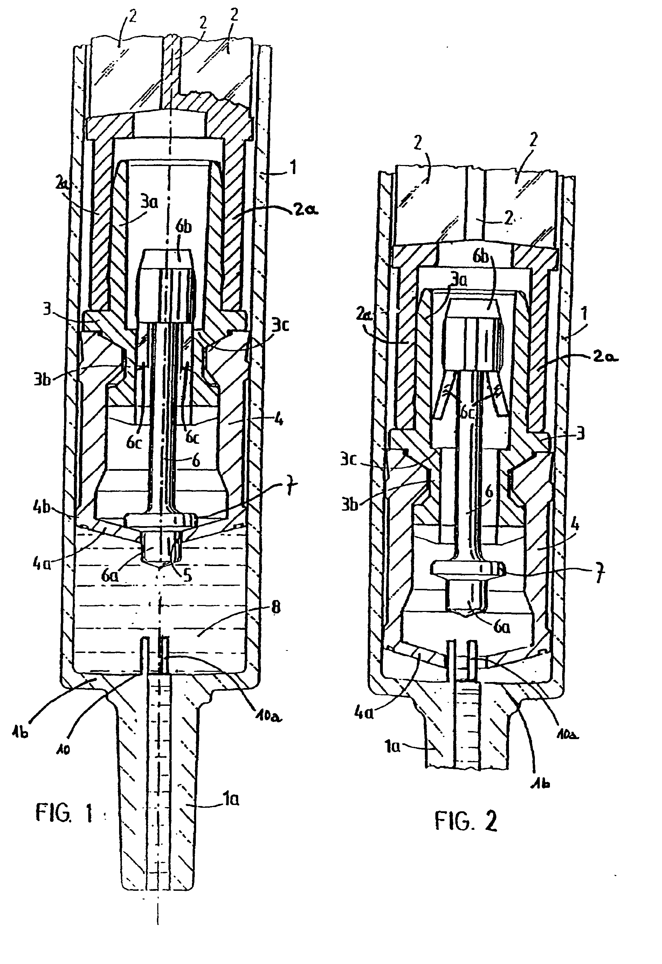

La seringue représentée aux figures 1 et 2 comprend un corps tubulaire 1, à l'extrémité duquel se trouve un embout la destiné à recevoir une aiguille hypodermique. Dans ce corps tubulaire coulisse un piston actionné à l'aide d'une tige 2. Cette tige, de forme cruciforme, est terminée par une pièce 2a ayant une forme de douille.

Le piston comporte une pièce intermédiaire 3, dont une partie supérieure 3a, en forme de manchon, est forcée par emboîtage axial dans la pièce 2a. Une pièce 4, en forme de douille, s'accroche à cran à la pièce intermédiaire 3. Cette pièce 4 présente, d'une part, un bourrelet extérieur 4b qui prend appui contre la face interne du corps tubulaire 1 afin d'assurer l'étanchéité entre le piston et le corps tubulaire et, d'autre part, un fond 4a qui se trouve au contact du liquide à injecter 8 et lui transmet la poussée du piston.

Le piston comporte une chambre interne dont l'espace est délimité par les faces internes des pièces 3 et 4 ainsi liées. La pièce intermédiaire 3 comporte une partie inférieure 3b délimitant un espace cylindrique inférieur et une partie supérieure 3a adjacente délimitant un espace cylindrique supérieur, le diamètre de l'espace cylindrique inférieur étant inférieur à celui de l'espace cylindrique supérieur de façon à constituer un épaulement 3c à la jonction des deux parties 3a et 3b. Des évents ont été pratiqués au sein de la pièce 2a afin d'équilibrer à la pression atmosphérique la pression régnant au sein de la chambre interne.

Le fond 4a de la pièce 4 est percé en son centre d'une ouverture 5 dans laquelle est engagée la partie terminale antérieure 6a, ci-après dénommée tête d'obturation 6a, d'une pièce d'obturation 6. Cette pièce d'obturation, de forme essentiellement cylindrique, est logée au sein de la chambre interne du piston, son axe de symétrie se superposant à celui de la seringue. La pièce d'obturation est montée de façon à pouvoir coulisser le long des parois de la partie inférieure 3a de la pièce intermédiaire. Elle se termine, à son extrémité postérieure, par une tête 6b présentant, dirigées vers l'avant, deux ailettes élastiques 6c, diamétralement opposées et maintenues, avant ou au cours d'une première injection, dans la position présentée à la figure 1. Les dimensions de la tête d'obturation 6a, de forme conique tronquée, sont telles qu'elle puisse se loger et coulisser au sein de l'ouverture 5 tout en assurant une parfaite étanchéité. Par exemple, dans le cas d'une seringue conçue pour permettre l'injection d'un volume maximal de 2,5 ml de liquide, l'ouverture a un diamètre de 2 mm, le cône tronqué de la tête d'obturation a une section inférieure dont le diamètre est également de 2 mm et une section supérieure dont le diamètre est de 2,5 mm. La tête d'obturation 6a est surmontée d'une couronne 7 qui fait saillie latéralement servant d'une part de butée pour le positionnement de la pièce d'obturation à l'intérieur de la chambre interne, et contribuant d'autre part à l'étanchéité de ladite chambre interne.

Le corps tubulaire 1 de la seringue comporte une contre-pièce 10 solidaire de son fond 1b, à proximité de l'embout 1a. Cette contre-pièce comporte des ergots 10a faisant saillie par rapport au fond 1b et dont la disposition est choisie de façon qu'ils se trouvent dans le prolongement de la tête d'obturation 6a de la pièce d'obturation, tout en laissant un passage suffisant au liquide à injecter. Cette contre-pièce sera présentée en détail plus loin.

Comme on le voit à la figure 1, les deux ailettes 6c prennent appui sur les parois de la partie inférieure 3a définissant l'espace cylindrique inférieur. Le diamètre de cet espace cylindrique est tel que les ailettes s'y trouvent repliées, sous contraintes, le long de la pièce d'obturation.

Lorsque le piston est mis en action, il exerce une pression sur le liquide contenu dans le corps qui est alors éjecté au travers de l'embout 1a.

Les forces de friction, s'exerçant à la fois sur les ailettes et sur la tête d'obturation 6a de la pièce d'obturation, sont telles que la pression que peut exercer le liquide sur cette tête ne soit pas suffisante pour faire coulisser la pièce d'obturation 6.

Lorsque le piston arrive en fin de course, la pièce d'obturation 6 entre en contact, par l'intermédiaire de sa tête 6a, avec les ergots 10a de la contre-pièce 10 qui joue alors le rôle de butée. En poursuivant sa course jusqu'à atteindre le fond 1b de la seringue, le piston s'empale sur la contre-pièce 10, ce qui a pour effet de chasser la tête d'obturation 6a hors de l'ouverture 5, la pièce d'obturation se déplaçant alors à l'intérieur de la chambre interne du piston, les ailettes 6c coulissant le long des parois de la partie inférieure 3b de la pièce intermédiaire 3, ces ailettes se déployant ensuite à l'intérieur de l'espace cylindrique supérieur constitué par la partie supérieure 3a de la pièce intermédiaire lorsque leurs extrémités dépassent l'épaulement 3c.

La pièce d'obturation se trouve alors dans la position représentée à la figure 2, les ailettes élastiques 6c, étant déployées dans une configuration qui leur est plus stable et prenant appui sur les parois de la partie supérieure 3a, se trouvent bloquées par l'épaulement 3c, rendant impossible le retour de la pièce d'obturation à sa position initiale, cette pièce n'obturant définitivement plus l'ouverture 5.

Il résulte de cet agencement qu'il n'est alors plus possible de réaspirer du liquide. En effet, par la libération de l'ouverture 5, l'intérieur du corps de la seringue communique avec la chambre interne du piston et se trouve à son tour équilibré à la pression atmosphérique. Le piston, par un mouvement de recul, n'est plus capable d'y créer une dépression.

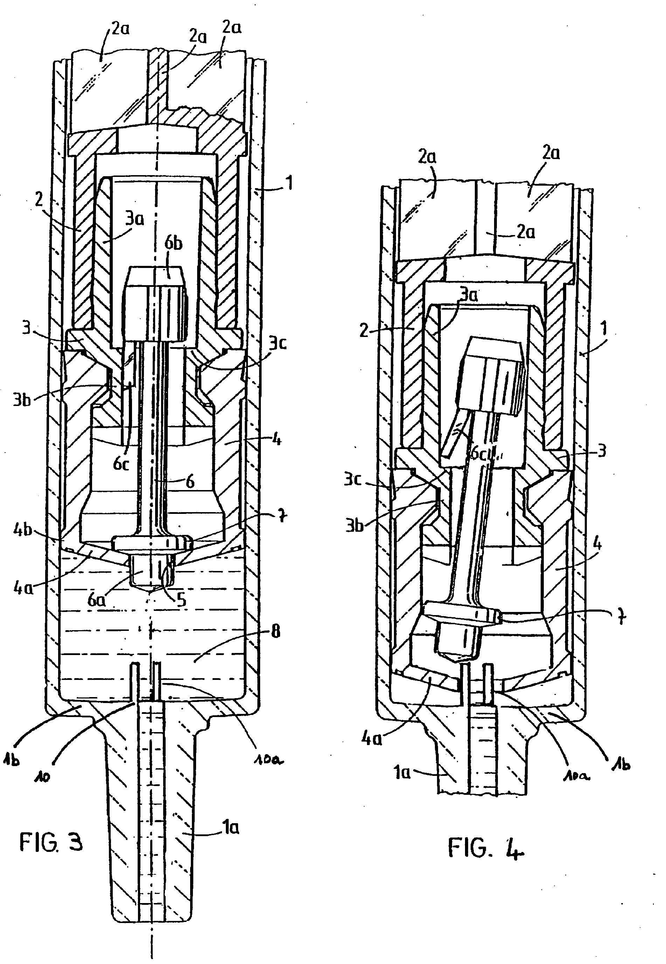

La variante représentée aux figures 3 et 4 ne diffère de la première forme d'exécution que par l'agencement de la pièce d'obturation 6. Dans cette variante, la pièce d'obturation 6 ne comporte qu'une seule ailette élastique 6c, de structure et de fonction toutefois identiques à celles des deux ailettes de la pièce d'obturation de la forme d'exécution précédente.

La présence de cette unique ailette confère à la pièce d'obturation une dissymétrie. La conséquence en est que, dès que la tête d'obturation 6a est forcée de s'éclipser de l'ouverture 5, la pièce d'obturation 6 pivote sur elle-même sous l'action de la poussée de l'ailette contre la paroi de la partie inférieure 3b. Comme le montre la figure 4, la pièce d'obturation perd alors définitivement son alignement avec l'ouverture 5 et n'est plus en mesure de l'obturer de nouveau.

Telle que représentée sur les figures 1 à 4, la contre-pièce 10 est constituée par trois ergots 10a qui font saillie sur le fond 1b du corps tubulaire, dans le prolongement des parois internes de l'embout 1a. La hauteur des ergots 10a est choisie de façon à provoquer un déplacement suffisant de la pièce d'obturation à l'intérieur de la chambre interne du piston, lorsque le piston arrive en fin de course, pour permettre le déploiement des ailettes 6c à l'intérieur de l'espace cylindrique supérieur constitué par la partie supérieure 3a de la pièce intermédiaire, comme décrit plus haut.

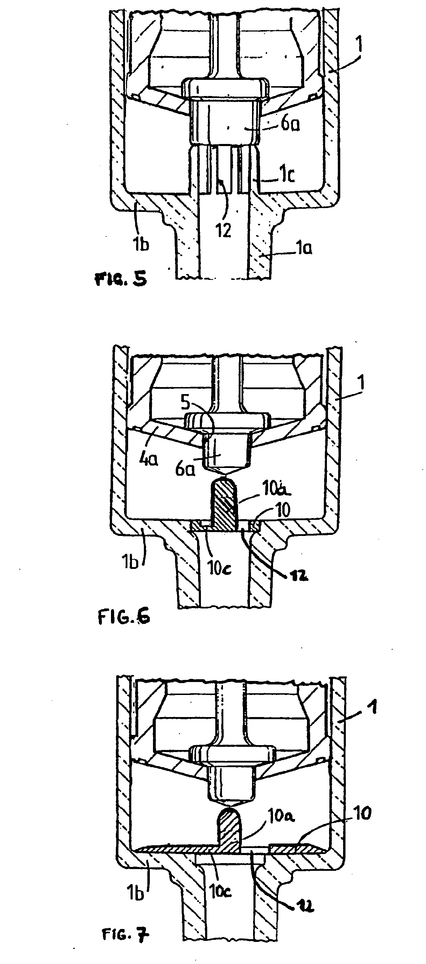

Selon la variante représentée par la figure 5, la contre-pièce 10 est constituée par une saillie annulaire 1c que présente en son centre le fond 1b du corps tubulaire de la seringue. Cette saillie 1c est crénelée, ce qui ménage des passages 12 permettant au liquide que contient la seringue de passer dans l'embout la alors même que la tête d'obturation 6a de la pièce d'obturation est en appui sur ladite saillie.

Dans la variante représentée sur la figure 6, le fond 1b du corps tubulaire 1 de la seringue est garni en son centre d'une rondelle 10b présentant des bras radiaux 10c aboutissant à un plot central ou à une borne 10a en forme d'ergot contre lequel vient buter la tête d'obturation 6a lorsque le piston arrive en fin de course. La taille de ce plot 10a est choisie de façon que l'on obtienne les effets précédemment décrits. La rondelle est sertie dans la masse du corps de seringue et s'appuie en partie sur l'ouverture de l'embout 1a. L'espacement entre les bras radiaux est suffisant pour ne pas entraver le passage du liquide à injecter.

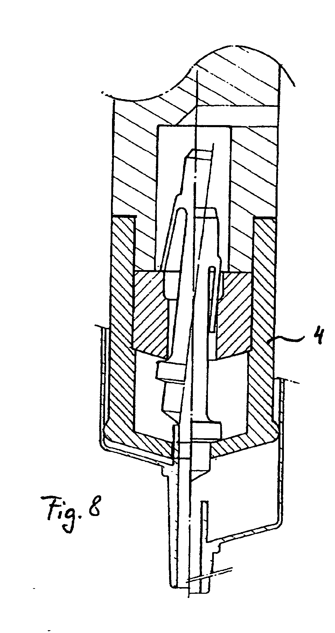

La variante présentée à la figure 7 diffère de la variante précédente par la forme de la rondelle centrale 10b, placée sur le fond 1b du corps tubulaire 1 de la seringue. Cette rondelle présente des bras radiaux 10c portant un plot central 10b contre lequel vient buter la tête d'obturation 6a lorsque le piston arrive en fin de course. Cette variante d'exécution présente l'avantage que la rondelle peut être disposée sur le fond de tout corps de seringue conventionnelle déjà existant.

Telle que représentée aux figures 1 à 4, la pièce 4 s'accroche à cran à la pièce intermédiaire 3. Afin de faciliter le montage, qui s'effectue par emboîtage axial, la pièce 4 est de préférence réalisée en une matière plastique semi-rigide ou souple, par exemple en caoutchouc synthétique.

Selon une variante d'exécution, représentée à la figure 8, la pièce 4 est réalisée en matière plastique rigide.

L'ensemble décrit et représenté est de fabrication aisée toutes les pièces pouvant être réalisées en matière plastique par un moulage simple et assemblées aisément par emboîtage axial, permettant une production industrielle à grande échelle, notamment une fabrication en série automatique, par exemple au moyen de carrousels automatiques puisque l'assemblage des différentes parties de la seringue ne nécessite aucun positionnement angulaire relatif. En outre, la conception de la seringue selon l'invention permet également l'utilisation, en les adaptant, des composants traditionnels des seringues dites "à trois corps", seringues constituées d'un cylindre, d'un piston et d'un joint.

De plus, le fonctionnement de la seringue selon l'invention est totalement fiable dès lors que le déplacement de la pièce d'obturation qui l'amène dans sa position éclipsée est provoqué uniquement lorsque le piston arrive en fin de course, ce qui permet, entre autres avantages, de pouvoir injecter l'intégralité du liquide contenu dans la seringue.

## Revendications

1. seringue médicale comprenant un corps tubulaire (1), dans lequel coulisse un piston (3, 4) comportant une ouverture (5), une pièce d'obturation (6) comportant à l'une de ses extrémités une tête d'obturation (6a) engagée dans l'ouverture (5) et obturant ladite ouverture avant usage de la seringue, ledit piston (3, 4) étant agencé de façon à constituer une chambre interne à l'intérieur de laquelle est logée la pièce d'obturation (6), ladite pièce d'obturation comprenant un organe (6c) agencé pour empêcher son retour à sa position d'obturation lorsqu'elle en a été chassée, ledit organe s'appuyant, avant injection, contre une paroi interne de ladite chambre interne, **caractérisée en ce que** la pièce d'obturation est agencée pour coopérer, lorsque le piston arrive en fin de course d'injection, avec une contre-pièce (10) solidaire du corps tubulaire de la seringue, de façon à chasser ladite pièce d'obturation hors de ladite ouverture (5), sans recourir à la déformation d'une membrane souple, que ladite pièce d'obturation présente des moyens de butée (7) agencés de façon à limiter la course de la pièce d'obturation à l'intérieur de ladite chambre interne, que l'ensemble formé par ledit piston et ladite pièce d'obturation est agencé de façon à engendrer une force de friction qui s'ajoute à celle qu'exerce le bord de l'ouverture (5) sur la tête d'obturation, et de façon que la pression du liquide s'exerçant sur la tête d'obturation pendant l'injection est insuffisante pour faire coulisser la pièce d'obturation le long de ladite paroi interne et chasser la tête d'obturation de l'ouverture (5), et **en ce que** toutes les pièces de la seringue sont conformées de façon à pouvoir être produites par moulage et montées en série automatiquement par emboîtage axial.

2. Seringue selon la revendication 1, **caractérisée en ce que** la chambre interne comprend une pièce intermédiaire (3) et une douille (4) accrochée à cran à la pièce intermédiaire, que la pièce d'obturation (6) est munie à sa partie postérieure d'éléments de verrouillage aptes à s'engager sur un épaulement intérieur (3c) de la pièce intermédiaire lorsque le piston atteint la fin de sa course, de sorte que les éléments de verrouillage sont placés en position active et que la tête d'obturation est surmontée d'une couronne 7 faisant saillie latéralement, formant une butée de positionnement de la pièce d'obturation à l'intérieur de ladite chambre interne.

3. Seringue selon la revendication 2, **caractérisée en ce que** les éléments de verrouillage comportent au moins une ailette tolérant une déformation élastique (6c), dont l'extrémité libre est orientée en direction du fond du corps de la seringue et coopère avec la paroi de la chambre interne, la paroi de la chambre interne étant conformée de façon à permettre à l'extrémité libre de l'ailette de coulisser contre la paroi de la chambre interne lorsque la pièce d'obturation se déplace à l'intérieur de la chambre après avoir buté contre la contre-pièce (10), mais à empêcher ensuite le coulissement de l'extrémité libre de l'ailette en sens inverse.

4. Seringue selon la revendication 3, **caractérisée en ce que** la chambre interne comporte un premier espace (3b) dont les dimensions sont choisies de façon à maintenir l'ailette (6c) repliée le long de la pièce d'obturation (6) lorsque la tête d'obturation (6a) de la pièce d'obturation obture l'ouverture (5), et un second espace (3a) dont les dimensions sont choisies de façon à permettre le déploiement de l'ailette lorsque la pièce d'obturation a été déplacée à l'intérieur du piston après avoir buté sur la contre-pièce (10).

5. Seringue selon la revendication 4, **caractérisée en ce que** le diamètre de la couronne (7) est choisi de sorte que celle-ci ne peut pas pénétrer dans le premier espace (3b).

6. Seringue selon l'une des revendications précédentes, **caractérisée en ce que** le dimensionnement des constituants de l'assemblage formé par le piston et la pièce d'obturation est tel que lors du montage, la pièce d'obturation peut s'engager axialement dans la pièce intermédiaire par sa tête (6b) munie d'éléments de verrouillage, que la douille peut être accrochée à la pièce intermédiaire après mise en place de la pièce d'obturation et que le dispositif ainsi assemblé peut être fixé au piston par engagement à force d'un manchon cylindrique (3a) de la pièce intermédiaire dans un alésage correspondant (2a) du piston.

7. Seringue selon l'une des revendications précédentes, **caractérisée en ce que** la contre-pièce (10) comporte au moins un organe (10a) faisant saillie par rapport au fond du corps tubulaire de la seringue.

8. Seringue selon l'une des revendications 1 à 7, **caractérisée en ce que** la contre-pièce (10) est constituée par au moins trois ergots (10a) qui font saillie sur le fond (1b) du corps tubulaire de la seringue, sur le pourtour de l'orifice destiné à l'injection du liquide contenu dans la seringue.

9. Seringue selon l'une des revendications 1 à 7, **caractérisée en ce que** la contre-pièce est constituée par une saillie annulaire crénelée (10a) bordant l'orifice destiné à l'injection du liquide contenu dans la seringue.

10. Seringue selon l'une des revendications 1 à 7, **caractérisée en ce que** la contre-pièce est constituée par une rondelle (10b) présentant des bras radiaux (10c) portant, au centre de ladite rondelle, une borne (10a) servant de butée pour la pièce d'obturation.

11. Seringue selon l'une des revendications 1 à 10, **caractérisée en ce qu'**elle comprend un dispositif de joint étanche (3, 4, 6) destiné à être associé au piston de la seringue, ledit dispositif de joint comportant une pièce d'obturation (6) qui en fin de course du piston provoque, en rencontrant une butée (10) placée au fond du corps de la seringue, une déformation irréversible du dispositif, de manière à exclure la possibilité d'une seconde utilisation de la seringue, la pièce d'obturation (6) étant montée dans un assemblage de guidage lié au piston, l'assemblage étant formé lui-même d'une pièce intermédiaire (3) et d'une douille (4) accrochée à cran à la pièce intermédiaire, et **en ce que** la pièce d'obturation (6) est pourvue à une extrémité d'une tête (6b) munie d'éléments de verrouillage (6c) aptes à s'engager sur un épaulement intérieur (3c) de la pièce intermédiaire et à l'autre extrémité d'une partie terminale (6a) qui en position active obture une ouverture (5) de la douille mais est repoussée par la butée en fin de course du piston, ce qui place les éléments de verrouillage en position active, et **en ce que** le dimensionnement des constituants de l'assemblage est tel que lors du montage, la pièce d'obturation peut s'engager axialement dans la pièce intermédiaire par sa tête (6b) munie d'éléments de verrouillage, la douille peut être accrochée à la pièce intermédiaire après mise en place de la pièce d'obturation et le dispositif de joint ainsi assemblé peut être fixé au piston par engagement à force d'un manchon cylindrique (3a) de la pièce intermédiaire dans un alésage correspondant (2a) du piston.

## Claims

1. Medical syringe comprising a tubular body (1), in which a piston (3, 4) slides, the piston having an opening (5), a blocking unit (6) having at one of its ends a blocking head (6a) engaged in the opening (5) and blocking said opening before the syringe is used, said piston (3, 4) being arranged in such a way as to constitute an inner chamber inside which the blocking unit (6) is housed, said blocking unit comprising an element (6c) arranged to prevent its returning to its blocking position once said blocking unit has been expelled thereof, said element resting prior to the injection against an inner wall of said inner chamber, **characterised in that** the blocking unit is arranged to cooperate, when the piston reaches the end of its path, with a counter-piece (10) integral with the tubular body of the syringe, so as to expel said blocking unit out of the opening (5), without using the deformation of a flexible membrane, **in that** said blocking unit is provided with stop means (7) arranged so as to limit the path of the blocking unit within said inner chamber, **in that** the assembly formed by said piston and said blocking unit is arranged in such a way as to generate a force of friction which is added to the force exerted by the edge of the opening (5) on the blocking head, and so that the pressure of the liquid acting on the blocking head during the injection is insufficient to cause the blocking unit to slide along said inner wall and to expel the blocking head out of the opening (5), and **in that** all the elements of the syringe are designed to be produced by moulding and assembled on an automatic production line by axial fitting.

2. Syringe according to claim 1, **characterised in that** the inner chamber comprises an intermediate element (3) and a socket (4) hooked with a notch to the intermediate element, **in that** the blocking unit (6) is provided at its rear section with locking elements capable of engaging on an inner shoulder (3c) of the intermediate element when the piston reaches the end of its path, thereby placing the locking elements in the active position and **in that** the blocking head is surmounted by a crown (7) which projects laterally, forming a stop for the position of the blocking unit inside said inner chamber.

3. Syringe according to claim 2, **characterised in that** the locking elements comprise at least one fin (6c) accommodating elastic deformation, whose free end is directed towards the base of the body of the syringe and cooperates with the wall of the inner chamber, said wall of the inner chamber being designed so as to allow the free end of the fin to slide against the wall of the inner chamber when the blocking unit moves inside the chamber after having come to a stop against the counter-piece (10) but to then prevent the sliding of the free end of the fin in the opposite direction.

4. Syringe according to claim 3, **characterised in that** the inner chamber comprises a first space (3b) whose dimensions are chosen in order to maintain the fin (6c) folded back along the blocking unit (6) when the blocking head (6a) of the blocking unit is blocking the opening (5) and a second space (3a) whose dimensions are chosen in order to allow the deployment of the fin when the blocking unit has been moved inside the piston after coming to a stop against the counter-piece (10).

5. Syringe according to claim 4, **characterised in that** the diameter of the crown (7) is chosen so that the crown can not enter into the first space (3b).

6. Syringe according to any one of the preceding claims, **characterised in that** the dimensioning of the components of the assembly formed by the piston and the blocking unit is such that, when mounting the assembly, the blocking unit can engage axially into the intermediate element via its head (6b) provided with locking elements, **in that** the socket can be hooked to the intermediate element after positioning of the blocking unit, and **in that** the joint device once so assembled can be fixed to the piston by forcing a cylindrical sleeve (3a) of the intermediate element in a corresponding bore (2a) of the piston.

7. Syringe according to any one of the preceding claims, **characterised in that** the counter-piece (10) comprises at least one element (10a) forming a projection in relation to the base of the tubular body of the syringe.

8. Syringe according to any one of claims 1 to 7, **characterised in that** the counter-piece (10) is made up of at least three lugs (10a) which project from the base (1b) of the tubular body of the syringe, on the periphery of the orifice intended for the injection of the liquid contained in the syringe.

9. Syringe according to any one of claims 1 to 7, **characterised in that** the counter-piece consists of a crenellated annular projection (10a) bordering the orifice intended for the injection of the liquid contained in the syringe.

10. Syringe according to any one of claims 1 to 7, **characterised in that** the counter-piece consists of a washer (10b) with radial arms (10c) having, in the centre of the aforesaid washer, a limit (10a) acting as a stop for the blocking unit.

11. Syringe according to any one of claims 1 to 10, **characterised in that** it comprises a seal joint device (3, 4, 6) intended to be coupled with the piston of the syringe, said joint device comprising a blocking unit (6) which causes, by coming up against a stop (10) placed in the bottom of the syringe body, when the piston reaches the end of its path, an irreversible deformation of the device, so as to exclude any possible re-use of the syringe, said blocking unit (6) being mounted into a guiding assembly coupled to the piston, wherein the assembly itself comprises an intermediate element (3) and a socket (4) hooked with a notch to the intermediate element, and **in that** the blocking unit (6) is provided at one end with a head (6b) provided with locking elements (6c) capable of engaging on an inner shoulder (3c) of the intermediate element and at the other end with a terminal part (6a) which, in active position, closes an opening (5) in the socket, but which is forced back by the stop when the piston reaches the end of its path, thereby placing the locking elements in the active position, and **in that** the dimensioning of the assembly components is such that, when mounting the assembly, the blocking unit can engage axially into the intermediate element via its head (6b) provided with locking elements, the socket can be hooked to the intermediate element after positioning of the blocking unit and the joint device once so assembled can be fixed to the piston by forcing a cylindrical sleeve (3a) of the intermediate element in a corresponding bore (2a) of the piston.

## Patentansprüche

1. Medizinische Spritze mit einem rohrförmigen Körper (1), in dem ein Kolben (3, 4) gleitet, der eine Öffnung (5) aufweist, mit einem Verschlussstück (6), das an einem seiner Enden einen Verschlusskopf (6a) aufweist, der in die Öffnung (5) eingreift und diese Öffnung vor dem Gebrauch der Spritze verschließt, wobei der Kolben (3, 4) so geformt ist, dass eine innere Kammer gebildet wird, in deren Innenraum das Verschlussstück (6) gelegen ist, wobei das Verschlussstück ein Mittel (6c) aufweist, das eine Rückkehr in dessen Verschlussposition verhindert, sobald es aus dieser herausgeschoben wurde, wobei dieses Mittel vor der Injektion an einer inneren Wand der internen Kammer anliegt, **dadurch gekennzeichnet, dass** das Verschlussstück ausgelegt ist, um, wenn der Kolben das Ende des Injektionsweges erreicht, mit einem Gegenstück (10) zusammenzuwirken, das mit dem rohrförmigen Körper der Spritze fest verbunden ist, derart, dass das Verschlussstück aus der Öffnung (5) herausgleitet, ohne eine Deformation einer weichen Membran zu verursachen, dass das Verschlussstück Anschläge (7) aufweist, um den Weg des Verschlussstückes in den Innenraum der internen Kammer zu begrenzen, dass die Anordnung aus dem Kolben und dem Verschlussstück so ausgebildet ist, dass eine Reibungskraft erzeugt wird, die derjenigen hinzugefügt wird, die der Rand der Öffnung (5) auf den Verschlusskopf ausübt, und derart dass der Druck der Flüssigkeit, der auf den Verschlusskopf während der Injektion wirkt, nicht ausreichend ist, um das Verschlussstück entlang der inneren Wand zu verschieben und den Verschlusskopf aus der Öffnung (5) zu treiben, und dass alle Teile der Spritze so ausgebildet sind, dass sie im Spritzguss hergestellt und in Serie automatisch nacheinander verpackt werden können.

2. Spritze nach Anspruch 1, **dadurch gekennzeichnet, dass** die interne Kammer ein Zwischenstück (3) und eine Hülse (4) aufweist, die in einer Kerbe in dem Zwischenstück eingehakt ist, dass das Verschlussstück (6) in seinem hinteren Bereich mit Riegelelementen ausgerüstet ist, die sich auf eine innere Schulter (3c) des Zwischenstückes abstützen können, wenn der Kolben das Ende seines Weges erreicht hat, derart, dass die Riegelelemente in einer aktiven Position platziert sind, und dass der Verschlusskopf am oberen Ende mit einem seitlich wegragenden Kranz (7) versehen ist, der einen Positionsanschlag des Verschlussstückes im Inneren der internen Kammer bildet.

3. Spritze nach Anspruch 2, **dadurch gekennzeichnet, dass** die Riegelelemente zumindest einen Flügel aufweisen, der eine elastische Deformation (6c) zulässt und dessen freies Ende in Richtung auf den Boden des Körpers der Spritze gerichtet ist und mit der Wand der internen Kammer zusammenarbeitet, wobei die Wand der internen Kammer so ausgebildet ist, dass das freie Ende des Flügels an der Wand der internen Kammer gleiten kann, wenn das Verschlussstück im Innenraum der Kammer versetzt wird, dass aber anschließend das Gleiten des freien Endes des Flügels im Gegensinne verhindert wird.

4. Spritze nach Anspruch 3, **dadurch gekennzeichnet, dass** die interne Kammer einen ersten Raum (3b) aufweist, dessen Dimensionen gewählt sind, um den Flügel (6c) längs des Verschlussstückes (6) angefaltet zu halten, wenn der Verschlusskopf (6a) des Verschlussstückes die Öffnung (5) verschließt, und einen zweiten Raum (3a) aufweist, dessen Dimensionen gewählt sind, um das Entfalten des Flügels zu erlauben, wenn das Verschlussstück im Innenraum des Kolbens versetzt worden ist, nachdem es an dem Gegenstück (10) angeschlagen hat.

5. Spritze nach Anspruch 4, **dadurch gekennzeichnet, dass** der Durchmesser des Kranzes (7) so gewählt ist, dass er nicht in den ersten Raum (3b) eindringen kann.

6. Spritze nach einem der vorhergehenden Ansprüche, **dadurch gekennzeichnet, dass** die Dimensionierung der Teile der Anordnung, die durch den Kolben und das Verschlussstück gebildet wird, so ist, dass während der Montage das Verschlussstück axial in das Zwischenstück mit seinem mit den Riegelelementen ausgerüsteten Kopf (6b) eingreifen kann, dass die Hülse in das Zwischenstück eingehakt werden kann, nachdem das Verschlussstück platziert worden ist, und dass die so zusammengesetzte Einrichtung mit dem Kolben durch Aufbringen einer Kraft einer zylindrischen Manschette (3a) des Zwischenstückes in einer dem Kolben entsprechenden Bohrung (2a) befestigt wird.

7. Spritze nach einem der vorhergehenden Ansprüche, **dadurch gekennzeichnet, dass** das Gegenstück (10) zumindest ein Mittel (10a) aufweist, das vom Boden des rohrförmigen Körpers der Spritze wegragt.

8. Spritze nach einem der Ansprüche 1 bis 7, **dadurch gekennzeichnet, dass** das Gegenstück (10) durch zumindest drei Vorsprünge (10a) gebildet ist, die von dem Boden (1b) des rohrförmigen Körpers der Spritze wegragen und um den Umfang der Öffnung angeordnet sind, die für die Injektion der in der Spritze enthaltenen Flüssigkeit bestimmt ist.

9. Spritze nach einem der Ansprüche 1 bis 7, **dadurch gekennzeichnet, dass** das Gegenstück durch einen ringförmigen gerippten Vorsprung (10a) gebildet ist, der die Öffnung begrenzt, die zur Injektion der in der Spritze enthaltenen Flüssigkeit bestimmt ist.

10. Spritze nach einem der Ansprüche 1 bis 7, **dadurch gekennzeichnet, dass** das Gegenstück durch eine runde Scheibe (10b) mit radialen Armen (10c) gebildet ist, die in ihrem Zentrum einen Vorsprung (10a) aufweist, der als Anschlag für das Verschlussstück dient.

11. Spritze nach einem der Ansprüche 1 bis 10, **dadurch gekennzeichnet, dass** sie eine Einrichtung zur engen Verbindung (3, 4, 6) aufweist, die dem Kolben der Spritze zugeordnet ist, wobei diese Verbindungseinrichtung ein Verschlussstück (6) aufweist, das am Ende des Weges des Kolbens beim Auftreffen auf einen Anschlag (10) am Boden des Körpers der Spritze eine irreversible Deformation der Einrichtung verursacht, derart, dass die Möglichkeit eines zweiten Gebrauches der Spritze ausgeschlossen ist, wobei das Verschlussstück (6) in einer mit dem Kolben verbundenen Führungsanordnung montiert ist und diese Anordnung aus einem Zwischenstück (3) und einer Hülse (4) gebildet ist, die in das Zwischenstück eingehakt ist, und dass das Verschlussstück (6) an einem Ende mit einem Kopf (6b) versehen ist, der mit Riegelelementen (6c) ausgerüstet ist, die sich auf eine innere Schulter (3c) des Zwischenstückes abstützen können, und an seinem anderen Ende mit einem Endbereich (6a) versehen ist, der in seiner aktiven Position eine Öffnung (5) der Hülse verschließt, jedoch durch den Anschlag am Ende des Weges des Kolbens zurückgestoßen wird, was die Riegelelemente in die aktive Position versetzt, und dass die Dimensionierung der Teile der Anordnung so ist, dass während der Montage das Verschlussstück axial in das Zwischenstück mit seinem Kopf (6b), der mit den Riegelelementen versehen ist, eingreifen kann, die Hülse in das Zwischenstück eingehakt werden kann, nachdem das Verschlussstück eingesetzt ist, und die so zusammengesetzte Verbindungseinrichtung mit dem Kolben durch Einsetzen der Kraft einer zylindrischen Manschette (3a) des Zwischenstückes in einer dem Kolben entsprechenden Bohrung (2a) befestigt werden kann.
